(19) Europäisches Patentamt
European Patent Office
Office européen des brevets

(11) Publication number: **0 411 593 A2**

(12) **EUROPEAN PATENT APPLICATION**

(21) Application number: **90114724.9**

(22) Date of filing: **31.07.90**

(51) Int. Cl.$^5$: **A61K 31/535**, A61K 31/495, A61K 31/445, C07D 221/22

(30) Priority: **02.08.89 JP 201535/89**

(43) Date of publication of application: **06.02.91 Bulletin 91/06**

(84) Designated Contracting States: **AT BE CH DE DK ES FR GB GR IT LI LU NL SE**

(71) Applicant: **KURARAY CO., LTD.**
**1621 Sakazu**
**Kurashiki-City Okayama Prefecture 710(JP)**

(72) Inventor: **Hosogai, Takeo**
**5-10-26 Shitte Chuoh, Kamisu**
**Kashima-gun, Ibaraki-pref.(JP)**
Inventor: **Yamahara, Johji**
**23-9 Takasago-cho**
**Ootsu-City, Shiga Prefecture(JP)**

(74) Representative: **Patentanwälte Deufel- Schön- Hertel- Lewald**
**Isartorplatz 6**
**D-8000 München 2(DE)**

# (54) Use of bis (aminothiocarbonyl) disulfide for treating ulcer.

(57) Use of bis(aminothiocarbonyl)disulfide compounds represented by the general formula:

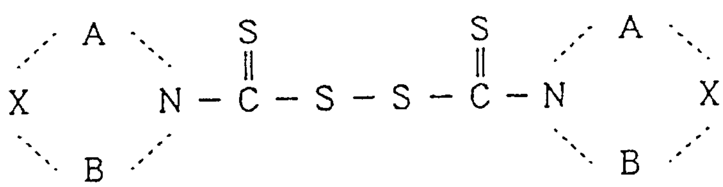

wherein

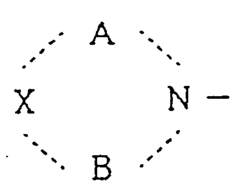

represents a saturated five to eight-membered heterocyclic group with or without being substituted, X represents a methylene group, a hydroxyalkylmethylene group having 2 to 5 carbon atoms, a hydroxyalkylimino group having 1 to 4 carbon atoms, or an oxygen atom, and A and B respectively represent an alkylene group having 0 to 7 carbon atoms with or without being substituted by a methyl group, for treating and/or preventing ulcer in human beings.

EP 0 411 593 A2

# USE OF BIS(AMINOTHIOCARBONYL)DISULFIDE COMPOUNDS FOR TREATING ULCER

## BACKGROUND FOR THE INVENTION

### Field of the Invention

The present invention relates to use of certain bis(aminothiocrbonyl)disulfide compounds for treating ulcer in human beings.

### Description of the Prior Art

In the compounds classified in bis(aminothiocarbonyl) disulfide, some of them are industrially applicable, for example, tetramethylthiuram disulfide as a vulcanization accelerator for rubber, tetraethylthiuram disulfide as a therapeutic agent for treating ethyl alcohol intoxication. However, it has been totally unknown that bis(aminothiocarbonyl)disulfides are effective as a therapeutic agent for peptic ulcer.

## DISCLOSURE OF THE INVENTION

It is an object of the present invention to provide use of certain compounds for treating ulcer in human beings.

The above object has been accomplished by use of bis(aminothiocarbonyl)disulfide compounds represented by the general formula (I):

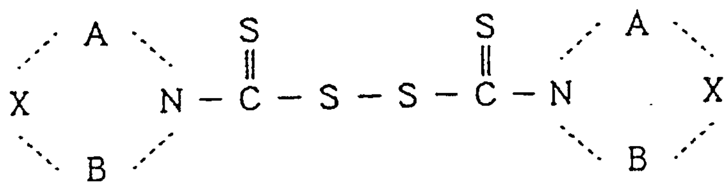

(I)

wherein

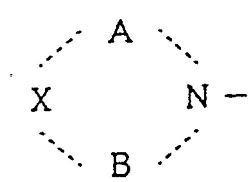

represents a saturated five to eight-membered heterocyclic group with or without being substituted, X represents a methylene group, a hydroxyalkylmethylene group having 2 to 5 carbon atoms, a hydroxyalkylimino group having 1 to 4 crbon atoms, or an oxygen atom, A and B represent respectively an alkylene group having 0 to 7 carbon atoms without or with being substituted by usually one methyl group or sometimes two or more methyl groups, for treating and/or preventing ulcer in human beings.

Another object of the invention is to provide use of bis(aminothiocarbonyl)disulfide compounds of the general formula (I) for preparaing pharmaceutical compositions for use in treating and/or preventing ulcer in human beings.

Other objects of the present invention will be apparent according to the following disclosure.

Examples of

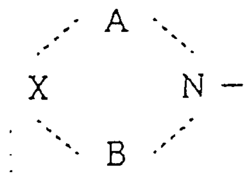

group in the formula (I) include pyrrolidino group, piperidino group, perhydroazepin-1-yl group, perhydroazocin-1-yl group, morpholino group, 2,6-dimethylmorpholino group, isoxazolidin-N-yl group; a piperidino group, a pyrrolidino group, a piperazino group, an imidazolidin-1-yl group, a pyrazolidin-1-yl group, a hexahydropyrimidin-1-y1 group and the like substituted by 2-hydroxyethyl group, 3-hydroxypropyl group, 2-hydroxypropyl group, 4-hydroxypropyl group and the like.

Examples of the general formula (I) are enumerated below:

Compound Number (1)

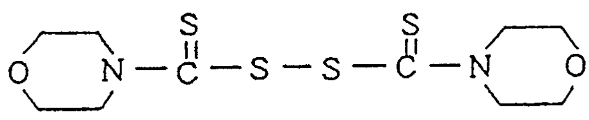

bis(morpholinothiocrbonyl)disulfide

Compound Number (2)

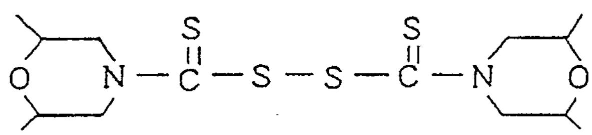

bis(2,6-dimethylmorpholinothiocarbonyl)disulfide

Compound Number (3)

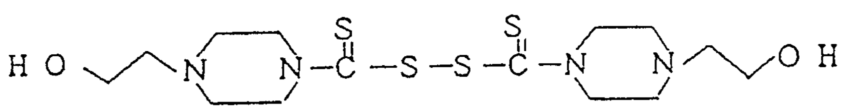

bis[[4-(2-hydroxyethyl)-1-piperadinyl]thiocarbonyl]disulfide

Compound Number (4)

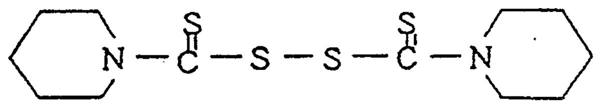

bis(piperidinothiocarbonyl)disulfide

Compound Number (5)

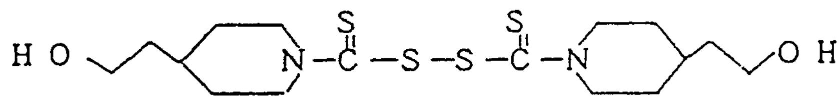

bis[[4-(2-hydroxyethyl)piperidino]thiocarbonyl]disulfide

The references describing the preparation methods of the Compound Number (1) to (4) are as follows, however, the Comppound Number (5) is a new compound.

Compound Number (1): J. J. D'Amico and E. Morita, Phosphorus Sulfur 3 , 255 (1977)
Compound Number (2): John H. Bigelow, U. S. Pat. No. 3,505,069
Compound Number (3): L.Florvall and H.Corrodi, Acta Pharm. Suecica, 7 , 7-22 (1970)
Compound Number (4): E. S. Blake, J. Am. Chem. Soc. 65 , 1267-9 (1943)

These compounds can be prepared by a similar method of the already known preparation process of bis(aminothiocarbonyl)disulfides (for example, E. E. Reid, Organic Chemistry of Bivalent Sulfur vol. IV, 246 (1962), Chemical Publishing Co., Inc., N.Y.), as shown in the reaction formula:

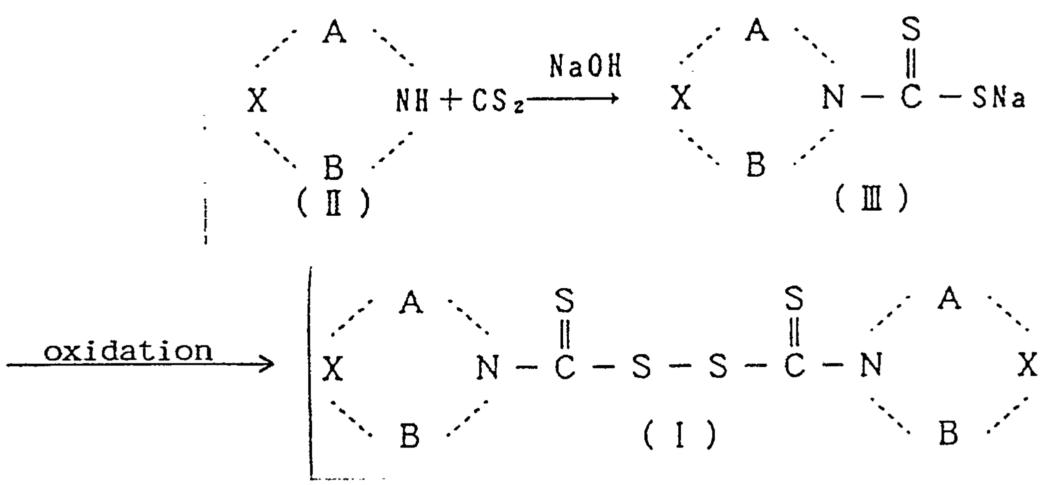


That is, to the cyclic amine shown as the formula (II) in the aforementioned reaction formula dissolved in a solvent such as methanol and ethanol, is added sodium hydroxide aqueous solution having one mole equivalent amount of (II), thereafter one mole equivalent of carbon disulfide is added in dropwise to the reaction mixture cooled under ice-cold water to obtain easily the sodium salt of dithiocarbamate of (III). The reaction product (III) is separated by filtration when the product is insoluble in the reaction solvent, or by the distillation of the reaction solvent when the product is soluble in the solvent.

Then the reaction product (III) is dissolved or dispersed in water, and oxidized with an oxidizing agent to obtain the product (I). As an oxidizing agent for the oxidation reaction, a wide variety of oxidizing agents such as a hydrogen peroxide aqueous solution, a sodium hypochlorite aqueous solution, iodine, bromine, chlorine and ammonium persulfate can be used.

The oxidation reaction of from (III) to (I) can be carried out by oxygen in the presence of a certain type of catalyst such as manganese acetate. The preferable reaction temperature for the oxidation reaction of from (III) to (I) is in the range of 0 ° C to 100 ° C. The product (I) which is usually a solid insoluble in water can be separated by filteration as a crude product.

The purification of the product (I) can be accomplished by recrystallizing with a proper solvent such as methanol, ethanol and chloroform, purifying with silica-gel column, or simply washing with a proper solvent.

Antiulcer pharmaceutical compositions referred to in the use of the present invention may be the form of tablets, capsules, powders, granules, electuaries, or liquid preparations such as sterile solutions or suspensions for oral or parenteral administration.

Tablets, granules and powders are suitable for orally administrating active ingredients of the present invention, and granules and powders can, if necessary, be formulated into capusules as a unit dose form.

Solid agents for oral administration may contain conventional excipients such as silicic anhydride, synthetic aluminium silicate, lactose, sucrose, corn starch, and microcrystalline cellulose, etc. binders such as gum arabic, gelatin, polyvinylpyrrolidone, etc.; lubricants such as magnesium stearate, talc, silica, etc. disintegrating agents such as potato starch, calcium carboxymethyl celulose, etc.; wetting agents such as polyethylene glycol sorbitan mono-oleate, sodium lauryl sulfate, etc. Tablets may be coated according to a conventional method.

Liquid preparations for oral administration may be aqueous or oily suspensions, solutions, syrups etc., or may be dried preparations which can be dissolved in a suitable vehicle prior to the use. Such liquid preparations may contain conventional emulsifiers such as lecithin, sorbitan mono-oleate, etc., emulsifying auxiliary agents such as sorbitol syrup, methyl cellulose, gelatin, etc.; non-aqueous vehicles such as coconut oil, peanut oil, etc.; antioxidants, coloring agents, flavoring agents, etc.

For use in parenteral administration, bis(aminothiocarbonyl)disulfide compounds of the general formula (I) may be dissolved or suspended in a sterile vehicle to obtain a liquid preparation. The preparation of the solution may be carried out by dissolving an active compound in a vehicle for injection, filtering the solution for sterility and pouring the solution into ampules. In the preparation, it is preferable to add adjuvants such as local anesthet ics, antiseptics and buffering agents in the vehicle.

The suspension can be prepared in the substantially the same manner as in the preparation of the solution except that an active compound is not dissolved but suspended in a vehicle and a procedure for sterility other than filteration is used.

Pharmaceutical compositions comprising bis(aminothiocarbonyl)disulfide compounds of the general formula (I) as an active ingredient are effective for treatment and/or prevention of ulcers of digestive organs, particularly stomach of human beings.

Although the effective amount or dose of the compounds depends on the extent of ulcers, the status of patients, kind of compounds of the general formula (I) to be used, proper doses generally range from about 100 to about 2500 mg per day for adult use.

## EXAMPLES

Examples of the present invention are described below.

### Example 1

Antiulcer activity (HCl-ethanol ulcer) 1.5 ml of 60% ethanol aqueous solution containing 150 mM of hydrochloric acid was orally administered to rats. One hour thereafter the rats were sacrificed and the length (mm) of HCl-ethanol induced ulcer generated at the mucosa of the stomach was measured. The sum of the ulcer length per animal is defined as ulcer coefficient. Specimens each was orally administered one hour before the HCl-ethanol administration. Inhibition rate was calculated by dividing the difference in ulcer coefficient between the control group and the specimen-administered group by the ulcer coefficient of the control group. The obtained results are shown in Table 1-1 and Table 1-2.

Table 1-1

| Test Example Number | Compound Number | Dose (mg/kg) | Animal Number | Inhibitation Rate (%) |
|---|---|---|---|---|
| | Control | -- | 8 | ---- |
| 1 | (1) | 5 | 6 | 93.7 |
| 2 | (2) | 5 | 6 | 81.7 |
| 3 | (3) | 5 | 6 | 24.3 |
| 4 | (4) | 5 | 6 | 64.1 |
| 5 | (5) | 5 | 6 | 37.4 |

Table 1-2

| Comparative Example No. | Compound | Dose (mg/kg) | Animal Number | Inhibiton Rate (%) |
|---|---|---|---|---|
| 1 | Spizofurone | 100 | 5 | 86.6 |
| 2 | Teprenone | 25 | 5 | 48.9 |

Spizofurone

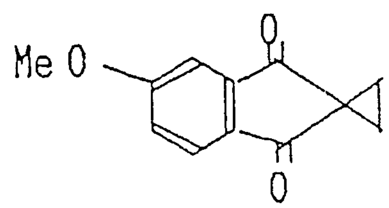

Teprenone

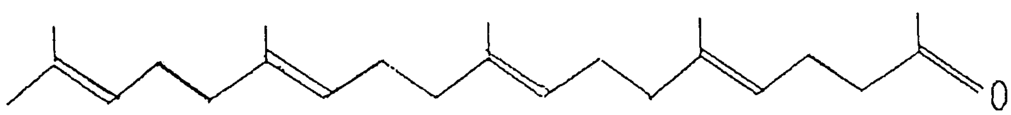

Both are typical antiulcer agents.

Example 2 Acute Toxicity

Acute toxicity tests through oral administration were carried out using male ICR-strain mice (5 weeks old).

The $LD_{50}$ values of the compound (1) of the present invention was 2000 mg/kg or more and thus higher saftety as compared with the effective amount was ascertained.

<u>Example 3</u> Drug suitable for oral administration

The following components were mixed and the mixture was formulated with tableting machine into

tablets.

| Component | Weight per tablet (mg) |
|---|---|
| Compound No. (1) | 100 |
| Corn starch | 50 |
| Crystalline Cellulose | 100 |
| Carboxymethyl cellulose | 50 |
| Total | 300 |

Example 4 Capsule for use in oral administration

The following components were mixed in a conventional manner and the mixture was filled into hard gelatin capsules.

| Component | Weight per capsule (mg) |
|---|---|
| Compound No. (1) | 50 |
| Neusilin® | 150 |
| Corn starch | 100 |
| Total | 300 |

Reference Example Synthesis of Compound No. (5)

16 mmol of 4-(2-hydroxyethyl)pyperidine was dissolved in 80 ml of anhydrous ethanol, and 1.28 g of 50% sodium hydroxide aqueous solution was added cooled under ice-cold water. Further, 0.9 ml of carbon disulfide was added and the reaction mixture was stirred for 2 hours at room temperature, thereafter ethanol as the solvent was distilled off by a rotary evaporator.

Crude sodium dithiocarbamate obtained by the above described reaction was dissolved in 50 ml of distilled water, and 10 ml of 12% sodium hypochlorite aqueous solution was added in dropwise for 20 min. After taking away of ice-cold water bath, the reaction mixture was stirred at room temperature for additional 2 hours, thereafter the formed precipitation was separated by filteration, and washed with anhydrous ethanol to obtain bis[[4-(2-hydroxyethyl)piperidiono]thiocarbonyl]disulfide (Compound Number (5)) having a melting point of 125-135 °C.

Fig. 1 represents infrared absorption spectrum of Compound Number (5) by KBr disc method.

## BRIEF DESCRIPTION OF THE DRAWINGS

Fig. 1 represents infrared absorption spectrum of bis [[4-(2-hydroxyethyl)piperidino]thiocarbonyl]disulfide by KBr disc method.

## Claims

1. Use of bis(aminothiocarbonyl)disulfide compounds represented by the general formula:

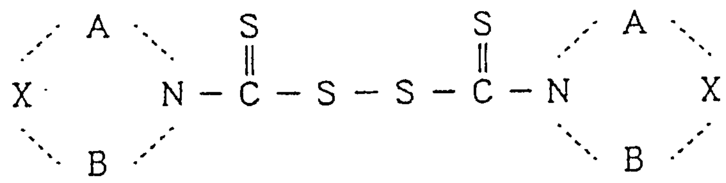

wherein

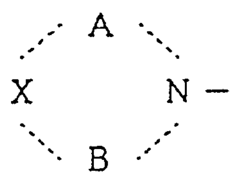

represents a saturated five to eight-membered heterocyclic group with or without being substituted, X represents a methylene group, a hydroxyalkylmethylene group having 2 to 5 carbon atoms, a hydroxyalkylimino group having 1 to 4 carbon atoms, or an oxygen atom, and A and B respectively represent an alkylene group having 0 to 7 carbon atoms with or without being substituted by a methyl group, for treating and/or preventing ulcer in human beings.

2. Use of bis(aminothiocarbonyl)disulfide compounds represented by the general formula:

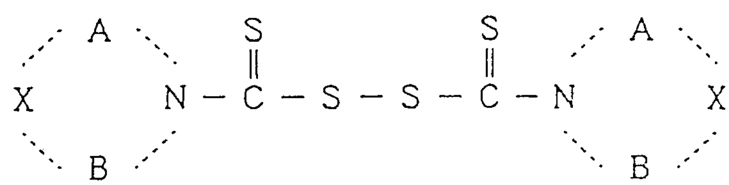

wherein

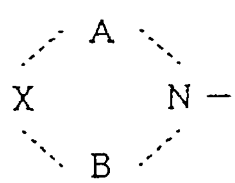

represents a saturated five to eight-membered heterocyclic group with or without being substituted, X represents a methylene group, a hydroxyalkylmethylene group having 2 to 5 carbon atoms, a hydroxyalkylimino group having 1 to 4 carbon atoms, or an oxygen atom, and A and B respectively represent an alkylene group having 0 to 7 carbon atoms with or without being substituted by a methyl group, for preparing pharmaceutical compositions for use in treating and/or preventing ulcer in human beings.

3. Use of claim 1 or 2 wherein the ulcer is an ulcer of digestive organs.

4. Use of claim 3 wherein the ulcer is the ulcer of stomach.

5. Bis[[4-(2-hydroxyethyl)piperidino]thiocrbonyl]disulfide.

Fig. 1

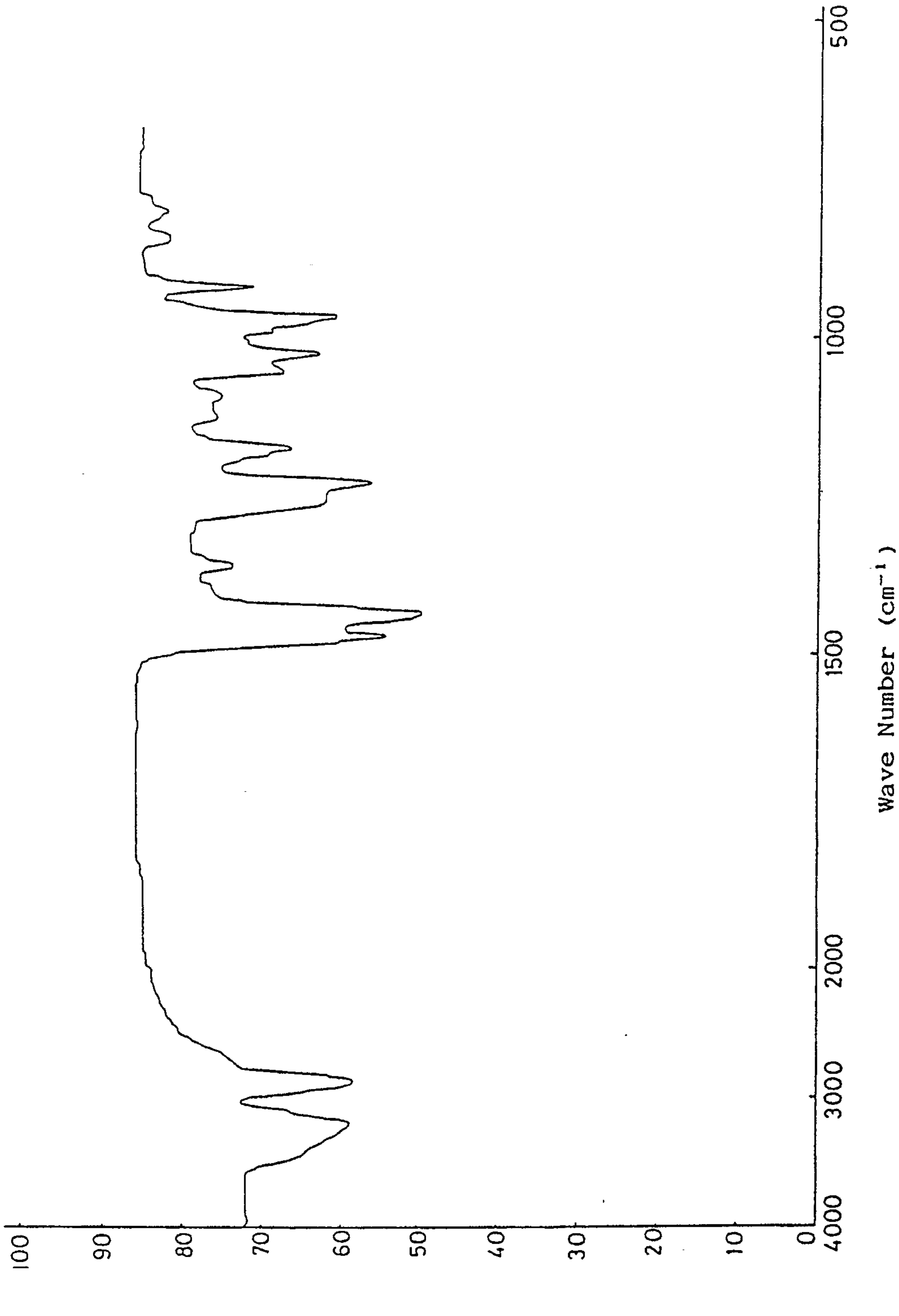


Applicant for Patent: Kuraray Co., Ltd.

Agent: Patent Attorney Katashi Honda